# EUROPEAN PATENT SPECIFICATION

(11) **EP 1 773 444 B1**
(45) Date of publication and mention of the grant of the patent: **20.09.2017**
(21) Application number: 05760247.6
(22) Date of filing: 09.06.2005
(51) Int. Cl.: A61M 37/00, A61B 17/20

(54) **PATCH APPLICATION DEVICE AND KIT**
VORRICHTUNG UND KIT ZUR PFLASTERAPPLIKATION
DISPOSITIF ET KIT D'APPLICATION DE PATCH

(30) Priority: 10.06.2004 US 578651 P
(43) Date of publication of application: 18.04.2007
(73) Proprietor: 3M Innovative Properties Company, St. Paul, MN 55133-3427 (US)
(72) Inventor: FREDERICKSON, Franklyn, L. 3M Center, Saint Paul, Minnesota 55133-3427 (US); CARTER, Chad, J. 3M Center, Saint Paul, Minnesota 55133-3427 (US); KUMAR, Kanta 3M Center, Saint Paul, Minnesota 55133-3427 (US); SEMONICK, Michael, A. 3M Center, Saint Paul, Minnesota 55133-3427 (US); WORTMAN, David, L., Saint Paul, MN 55133-3427 (US); WIRTANEN, David J., Saint Paul, MN 55133-3427 (US)
(74) Representative: Vossius & Partner Patentanwälte Rechtsanwälte mbB
(86) International application number: PCT/US2005/020283
(87) International publication number: WO 2005/123173

(56) References cited:
- WO-A-02/30300
- US-B1- 6 589 202

## Description

The present invention relates to the field of patch application devices, and in particular patch application devices for delivery of microneedle patches.

### BACKGROUND

Only a limited number of molecules with demonstrated therapeutic value can be transported through the skin, even with the use of approved chemical enhancers. The main barrier to transport of molecules through the skin is the stratum corneum (the outermost layer of the skin).

Devices including arrays of relatively small structures, sometimes referred to as microneedles or micro-pins, have been disclosed for use in connection with the delivery of therapeutic agents and other substances through the skin and other surfaces. The devices are typically pressed against the skin in an effort to pierce the stratum corneum such that the therapeutic agents and other substances can pass through that layer and into the tissues below.

Microneedle arrays may be used in conjunction with an applicator device capable of being used a number of different times. Unlike the applicator, the microneedle arrays are generally used once and then disposed of. The arrays are typically manufactured in a flat sheet-like configuration and temporarily attached to the applicator using, for example, an adhesive that permits the array to be detached from the applicator using a tweezers or the like. The process of applying an array can be very tedious and slow and may create a risk of damaging the numerous structures in the array. Additionally, the process of removing the array from the applicator requires a technician or other person to handle and dispose of the used array, creating handling issues to ensure that the risk of cross-contamination is minimized.

Issues associated with microneedle or microarray devices include the ability to effectively pierce the stratum corneum. That ability can be compromised by the desire to limit the height of the microneedle structures to avoid stimulating the nerves located under the stratum corneum. As a result of the limited height of the structures, it may be difficult to reliably pierce the stratum corneum in enough locations to effectively deliver a therapeutic agent to a patient.

WO 02/30300 A2 discloses a retainer that is provided for holding and application of a microprotrusion member to the stratum corneum with an impact applicator. The microprotrusion member includes a plurality of microprotrusions which penetrate the stratum corneum.

### SUMMARY OF THE INVENTION

The present invention provides a patch application device, a cartridge for use with an applicator in a patch application device, and a patch application kit. In particular, it has been found that an adhesive on the front surface (e.g., microneedle side) of the patch can be beneficially used to retain the patch in position within the applicator device until actuated. This can simplify manufacturing, as well as avoid the need to use adhesive on the back surface of the patch or use other more elaborate measures to retain the patch in position within the applicator.

An additional benefit of the present invention is the easy release of a patch from the applicator, allowing for consistent application of the patch to the skin surface, without compromising a substantial amount of energy and force used to apply the array.

Accordingly, in one embodiment of the present invention, a patch application device is provided that includes: a collar comprising an inner and outer surface, wherein at least one obstruction extends from the inner surface, and wherein the collar further comprises a proximal end and a distal end;
a patch within the collar comprising a distal-facing surface wherein at least a portion of the distal-facing surface of the patch comprises an adhesive, which adheres the patch to at least one obstruction; an applicator adjacent to the proximal end of the collar, the applicator comprising: a first stored position wherein at least a portion of the distal-facing surface of the patch is protected from external contact; a second released position wherein the patch is released from contact with the at least one obstruction in the collar and expelled from the collar; and a drive mechanism wherein the patch is forcibly expelled toward the distal end of the collar. The patch comprises a microneedle array and a backing. The microneedle array is manufactured of thermoplastic polymeric matierals.

In another aspect, a cartridge for use with an applicator in a patch application device comprising: a collar comprising: an inner and outer surface, wherein at least one obstruction extends from the inner surface; a proximal end and a distal end, wherein the proximal end of the collar is configured such that it may be attached and detached from a patch applicator device; a patch within the collar comprising a distal-facing surface wherein at least a portion of the distal-facing surface of the patch comprises an adhesive, which adheres the patch to at least one obstruction and further wherein the patch may be released from contact with at least one obstruction and expelled from the collar.

An applicator and cartridge according to the invention can be provided together as a kit. There is also provided a method of applying a patch using the forgoing device, cartridge, or kit.

In many instances the patch will be a microneedle array. Such arrays may be configured to penetrate the stratum corneum upon impact. The microneedle array can be of different sizes. In some cases, it will have a surface area of 5 square centimeters or less. Sometimes the array will be 1 square centimeter or less. The microneedle can include at least one microneedle with a height above a first major surface and a maximum base dimension, the height and the maximum base dimension ratio defining an aspect ratio, wherein the aspect ratio is 2:1 or more. At least one or more of the microneedles may be formed of one or more polymers. In some cases, the base of at least one microneedle may have an area of 900 square micrometers or more. Also, at least a portion of the microneedle array can comprise a non-patterned substrate.

The drive mechanism can comprise a piston and a driver operably connected to the piston, the driver providing acceleration of the piston. The piston may in some cases be selected to have a mass of about 8 grams or less, in some instances 4 grams or less, and in other instances a mass of 2 grams or less.

The device can be designed if desired so that microneedle patch is driven at a minimum velocity of 6 meters per second or more, and in some instances, a minimum velocity of 4 meters per second or more, but can also be limited if desired to a maximum velocity of 8 meters per second or less and in some instances, a maximum velocity of 10 meters per second or less. Delivering the microneedles at certain velocities can enhance perforation of the stratum corneum while limiting the sensation of pain experienced at the delivery site. The delivery device may use components with limited mass to reduce the tendency of the apparatus to stimulate nerves during delivery of patch application devices.

In operation, a microneedle patch cartridge can be attached to the applicator device prior to use and then detached from the applicator after use without the need to handle the array directly. The construction of the applicator device thus avoids the need for tweezers or similar tools in the attachment/detachment process.

As used herein, certain terms will be understood to have the meaning set forth below:
"Array" refers to the medical devices described herein that include one or more structures capable of piercing the stratum corneum to facilitate the transdermal delivery of therapeutic agents or the sampling of fluids through or to the skin.

"Microstructure," "microneedle" or "microarray" refers to the specific microscopic structures associated with the array that are capable of piercing the stratum corneum to facilitate the transdermal delivery of therapeutic agents or the sampling of fluids through the skin. By way of example, microstructures can include needle or needle-like structures as well as other structures capable of piercing the stratum corneum.

"Cartridge" refers to the combination of the collar component and the patch described herein. The cartridge is capable of being removed and/ or replaced to allow application of a patch to a target surface, such as a skin surface, to facilitate the transdermal delivery of therapeutic agents or the sampling of fluids through or to the skin.

The features and advantages of the present invention will be understood upon consideration of the detailed description of the preferred embodiment as well as the appended claims. These and other features and advantages of the invention may be described below in connection with various illustrative embodiments of the invention. The above summary of the present invention is not intended to describe each disclosed embodiment or every implementation of the present invention. The Figures and the detailed description which follow more particularly exemplify illustrative embodiments.

### BRIEF DESCRIPTION OF THE DRAWINGS

Figure 1 is a perspective view of a patch application device according to the present invention;
Figure 2 is a cross-sectional view of the application device of Figure 1 in the first stored position according to the present invention;
Figure 3 is a cross-sectional view of one embodiment of the collar of the application device of Figure 1 in the first stored position according to the present invention;
Figure 4 is a cross-sectional view of one embodiment of the collar of the application device of Figure 1 in the second released position according to the present invention;
Figure 5 is a graph of velocity (vertical axis) versus displacement of piston (horizontal axis) for an applicator device according to the present invention;
Figure 6 is a graph of velocity (vertical axis) versus displacement of piston and patch (horizontal axis) for an applicator device according to the present invention.
Figure 7 is a photomicrograph of a microneedle array according to the present invention; and
Figure 8 is a perspective view of a patch according to the present invention.

While the above-identified drawing figures set forth several embodiments of the invention, other embodiments are also contemplated, as noted in the discussion. In all cases, this disclosure presents the invention by way of representation and not limitation. It should be understood that numerous other modifications and embodiments can be devised by those skilled in the art, which fall within the scope and spirit of the principles of the invention. The figures may not be drawn to scale. Like reference numbers have been used throughout the figures to denote like parts.

### DETAILED DESCRIPTION OF ILLUSTRATIVE EMBODIMENTS OF THE INVENTION

The present invention provides patch application devices that may be useful for a variety of purposes. For example, the patch application devices may be used to deliver a patch to a skin surface in order to pierce the stratum corneum at a delivery site on a patient's skin. For example, the patch application device may be used to deliver drugs (including any pharmacological agent or agents) through the skin in a variation on transdermal delivery, or to the skin for intradermal or topical treatment, such as vaccination.

In some embodiments, the patch application device may be used in conjunction with microneedles to effectively deliver therapeutic drugs. Patch application devices of the present invention may have utility for a number of drugs and therapeutic indications. In one aspect, drugs that are of a large molecular weight may be delivered transdermally. Increasing molecular weight of a drug typically causes a decrease in unassisted transdermal delivery. Patch application devices of the present invention have utility for the delivery of large molecules that are ordinarily difficult to deliver by passive transdermal delivery. Examples of such large molecules include proteins, peptides, nucleotide sequences, monoclonal antibodies, DNA vaccines, polysaccharides, such as heparin, and antibiotics, such as ceftriaxone.

In another aspect, patch application devices of the present invention may have utility for enhancing or allowing transdermal delivery of small molecules that are otherwise difficult or impossible to deliver by passive transdermal delivery. Examples of such molecules include salt forms; ionic molecules, such as bisphosphonates, preferably sodium alendronate or pamedronate; and molecules with physicochemical properties that are not conducive to passive transdermal delivery.

In another aspect, patch application devices of the present invention may have utility for enhancing delivery of molecules to the skin, such as in dermatological treatments, vaccine delivery, or in enhancing immune response of vaccine adjuvants. In one aspect, the drug may be applied to the skin (e.g., in the form of a solution that is swabbed on the skin surface or as a cream that is rubbed into the skin surface) prior to applying a microneedle array.

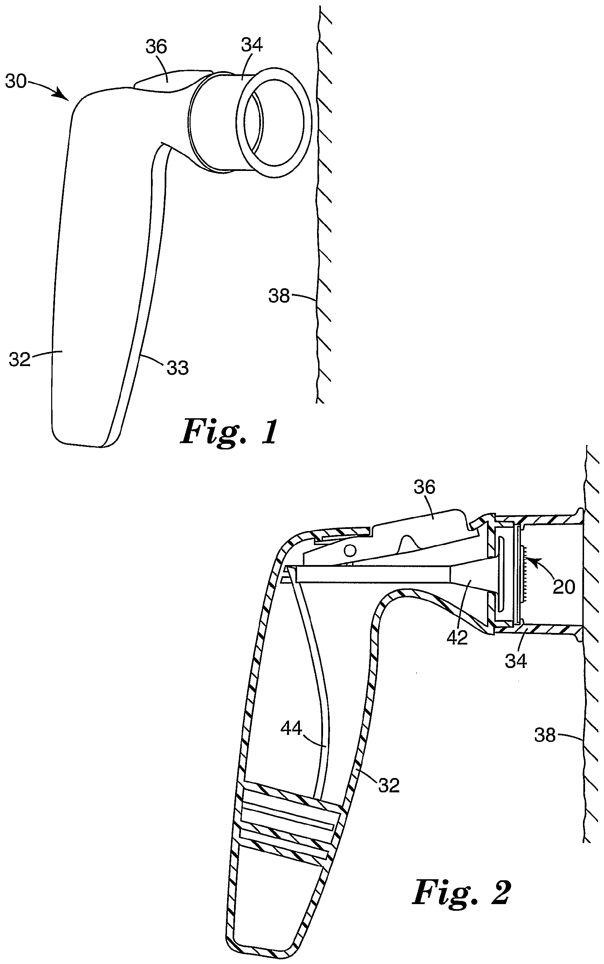

Referring now to Figure 1, one embodiment of the present invention provides a patch application device 30 including a left housing 32 and a right housing 33 fused together. The patch application device 30 provides a drive mechanism that when activated expels a patch 20 from the distal end of the collar 34.

Referring to Figure 2, the patch application device 30, shown in a first stored position, includes a piston 42 located within the left housing 32 and right housing 33. A driver 44 is operably connected to the piston 42 wherein the driver has stored energy, wherein release of the stored energy results in acceleration of the piston 42 toward an application surface 38 at a desired velocity.

The driver 44 may be provided by any mechanism capable of providing acceleration sufficient to reach the desired velocities as discussed herein. For example, the driver 44 may be in the form of a mechanical spring (e.g., a coil spring, leaf spring, etc.), compressed resilient member (e.g., rubber, etc.), compressed fluids (e.g., air, liquids, etc.), piezoelectric structure, electromagnetic structure, hammer device, etc. Regardless of the precise form of the driver 44, it should be capable of storing sufficient energy to accelerate the mass of the piston 42 and patch 20.

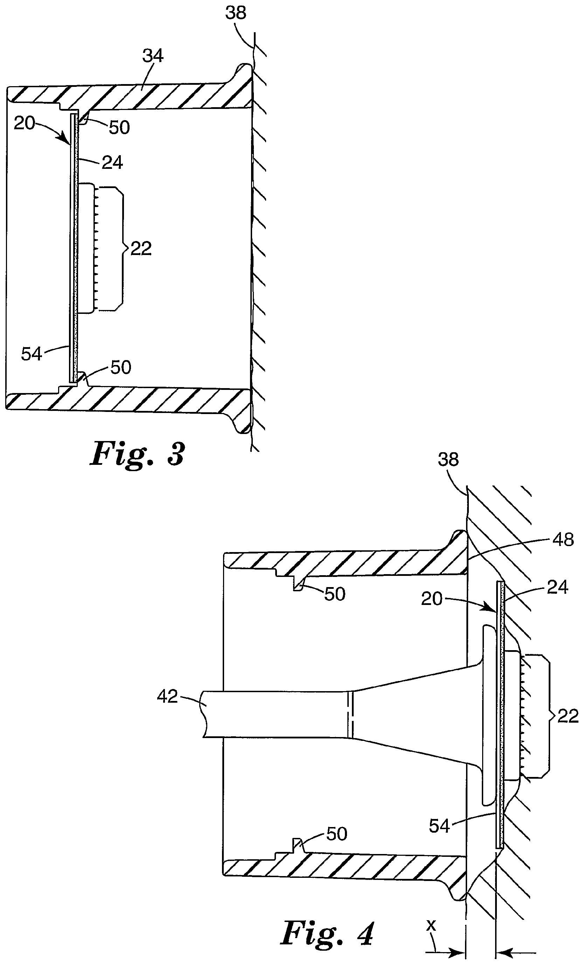

As shown in FIG. 2, the device is in a first stored position, the actuator 36 of the patch application device 30, has not been engaged, that is, the actuator has not been depressed. The driver 44 has stored energy and the piston 42 is not in contact with the patch 20, which is retained within the collar 34 of the device 30. In the second released position, shown in FIG. 4, the actuator 36 is engaged, allowing the driver 44 to urge the piston 42 towards the patch 20. The piston 42 breaks the adhesive bond between the patch 20 and the obstructions 50 in the collar 34 of the applicator device 30. The driver 44 is shown as a conventional leaf spring positioned partially within the hollow element and in sound communication with the left housing 32 and right housing 33 and operably connected to the piston 42. The actuator 36 is operably connected to the driver 44 and the piston 42 such that movement of the actuator 36 causes the driver to engage and urge the piston 42 to release a patch 20 from the distal end of the collar 34 and onto the application surface 38. One skilled in the art will appreciate that the piston 42 is sized to allow for movement between through the collar 34 without interfering with the obstructions in the collar.

Referring to Figures 3 and more specifically Figure 4, another feature depicted in connection with patch application device 30 is a collar 34 having an open distal end and a proximal end that may be engaged with the applicator housing 32, 33. The collar 34 is preferably placed in contact with the skin surrounding an application surface 38 during use of the apparatus 30. By forcing the collar against the skin, tautness of the skin within the delivery site may be increased which may have a beneficial result in perforation of the stratum corneum. The inner surface of the collar 34 provides one or more obstructions 50 which protrude from the internal walls of the collar 34. The patch 20 is contained in the collar 34 by adhesion to the one or more obstructions 50 in the inner surface of the collar 34. Although the depicted collar 34 is circular and would provide continuous contact about the periphery of a delivery site, it will be understood that collars used in connection with the present patch application device could be provided in a variety of shapes and configurations. For example, the collar may be discontinuous, that is, it may include gaps about the periphery of a delivery site.

The collar should have a sufficient height (i.e., the distance between the distal and proximal ends of the collar) to allow a patch to be contained and protected within the collar. The height of the collar will typically be about 5 cm or less, often about 2.5 cm or less, and sometimes about 1.0 cm or less. The obstructions (i.e., the portion of the obstructions that contact the patch adhesive) are spaced a sufficient distance away from the distal end of the collar in order to protect the patch from external contact. This distance may vary depending on a number of factors, such as the size and nature of the microneedle array, the type of surface to which the patch is to be applied, the force with which the collar is pressed against the application surface, and the desired velocity of the patch upon contact with the application surface. The obstructions will be spaced away from the distal end by a distance equal to or less than the height of the collar, typically by a distance of 2.0 cm or less, often by a distance of 1.0 cm or less, and sometimes by a distance of 0.6 cm or less. The collar will most typically have a inner cross-sectional area slightly larger than the area of the patch, so as to allow the patch to freely move from the collar when expelled by the drive mechanism. The relative size of the patch to the inner cross-sectional area of the collar may vary depending on the patch construction and the intended use of the device. In one embodiment, the patch diameter will be between about 1 and 5 mm smaller, and sometimes between about 2 and 3 mm smaller, than the inner diameter of the collar.

Each obstruction 50 is configured to hold and retain the patch 20 previously described herein. The obstructions 50 are dimensioned so that the patch 20 is retained within the internal surface of the collar 34. The obstructions 50 are in contact with the adhesive 24 on the distal-facing surface of the patch 20. In the second released position, shown in FIG. 4, the patch 20 is pushed past the obstructions 50 through the inner surface of the collar 34 and onto the application surface 38 where the adhesive adheres to the application surface 38. The number of obstructions 50 may vary from one to fifty. In some cases, it may be preferred that there are at least three and less than six obstructions, more preferably four obstructions in the inner surface of the collar 34.

The adhesive 24 serves a dual purpose. That is, the adhesive 24 serves the function of retaining the patch within the collar of the application device for storage, shipment, transport, and/or handling. Additionally, the adhesive 24 adheres to an application surface, such as the skin, when the patch is expelled from the device in the second released position. In some embodiments, the adhesive is a pressure sensitive adhesive. In other embodiments, the adhesive can be a medical grade adhesive. In some embodiments, the adhesive may be waterproof or water-resistant so that the patch remains adhered for a desired time period to a surface that is wet, contains moisture, or otherwise comes into contact with water during the time period that the patch is adhered to the surface. In one embodiment, the bond formed between the adhesive and the surface is not permanent, so that the patch can be removed when desired. When the adhesive is to be applied to a living organism or host, the adhesive should also be compatible with the host so that undue irritation at the treatment site is avoided.

Examples of suitable pressure-sensitive adhesives that may be used include acrylate and/or methacrylate polymers and copolymers, silicones, polyisobutylenes, synthetic rubbers, such as styrene-isoprene-styrene,and mixtures and blends of the foregoing. A particularly beneficial class of adhesives is the class of (meth)acrylate polymers, preferably acrylate embodiments thereof, suitable for use as pressure sensitive adhesives (PSAs). Representative embodiments of such (meth)acrylate PSAs are described in U.S. Pat. Nos. 4,693,776 (Krampe et al.); 4,751,087 (Wick); 4,737,577 (Brown); and Re 24,906 (Ulrich);

The bond between the adhesive of the patch and the obstructions of the collar may vary depending on a number of factors, including the size and shape of the obstructions, the contact area between the adhesive and the obstructions, the adhesive properties, the material composition of the obstructions, the surface treatment of the obstructions (if any), the amount of pressure used to secure the patch to the obstructions, or the storage conditions. The bond between adhesive and obstructions is preferably chosen so that it is high enough to prevent accidental dislodgement of the patch during storage and handling, but low enough to allow complete and easy release of the patch during application.

In one embodiment of this invention, the obstructions can be treated with a release coating material prior to attaching the patch to the obstructions. In another embodiment, release coating may be applied to the entire collar such as by dip coating the whole collar in a release coating solution, or by applying the coating solution only to the inner surface of the collar including protrusions. In another embodiment, the release coating can be applied to or extruded toward the inner surface of the collar including protrusions during the extrusion/manufacturing process of the collars. In one aspect, a release coating may prevent the patch from remaining adhered to one or more obstructions after the piston contacts the patch thus allowing the patch to release completely and easily from the collar. Examples of suitable release coatings include silicones, such as polydimethylsiloxane (US 3, 061,567, US4, 216,252, US 4,386,135 and references included therein,); Epoxysiloxanes (WO 81/02424); fluorocarbon polymers; fluorosilicones; composite flurochemical-silicone relelase coating (US 4, 171,397): silicone-organic hybrids, such as silicone-polyureas, silicone acrylates, polydimethylsiloxane-grafted copolymer (US 4,728,571); polyurethanes (US 3,342,625), and polymers having long alkyl side chains, which typically consists of a copolymer of an acrylic ester, vinyl ether, and acrylamide derivatives or the like. Silicones are preferred release coating materials which may be delivered from a solvent or a combination of solvents or coated as 100 percent solids. Silicones can be cured by condensation such as those described in US 4,530,882 and US 4,525,566, and D. R. Thomas in "Siloxane Polymer", S. J. Clarson, J. A. Semlyen, Eds. PTR Prentice Hall, Englewood Cliffs, NJ. 1993, or by radiation as such as described in US 3,655,713. Additional release coatings include UV cured silicones, such as UV9500 and UV9390C (both available from GE Silicones) or thermally cured silicones, such as Syl-off® 292, Syl-off® 291, Syl-off® 294, Syl-off® 7075 (available from Dow Corning)., or addition cure silicones Syl-off® 7010, Syl-off® 7020, Syl-off® 7395 (also available from Dow corning) In one embodiment, a mixture of 10 parts Syl-off® 292, 10 parts Syl-off® C4-2109 (release additive), 1.07 parts Syl-off® 297 (anchorage additive), 0.56 parts Syl-off® C4-2117 (fast cure additive), and 0.85 parts Dow Corning® 176 catalyst in a mixture of 91 parts heptane and 23 parts methyl ethyl ketone may be used as a release coating solution that can be cured at 150 °C for 2 minutes to provide a suitable release coating. In one embodiment, the peak force required to push a patch past the obstructions with a cylindrical probe is less than 400 grams and in some instances may be less than 200 grams.

In another embodiment of the present invention it may be desired that when actuated, the piston 42 reaches a distance x away from the distal end 48 of the collar 34. For example, this may allow the patch application device 30 to achieve improved patch adhesion upon the application surface 38 when the piston 42 travels beyond the distal end 48 of the collar 34. For example, it may be preferred that after actuation the distance x between the distal end 48 and the patch 20 is between about 0.000 inches (0.00 cm) and about 0.500 inches (1.27 cm), more preferably between about 0.050 inches (0.127 cm) and about 0.250 inches (0.635 cm), most preferably about 0.125 inches (0.318 cm).

The patch application device according to the present invention may include a piston designed to deliver microneedles at velocities that may enhance perforation of the stratum corneum while limiting the sensation of pain experienced at the delivery site. To accomplish this goal, the patch application device may use movable components with limited mass to reduce the tendency of the apparatus to stimulate nerves during delivery of the therapeutic drugs using the patch application device. In addition, the delivery apparatus includes a collar that, in one embodiment, may be forced against the skin around the patch application device to increase reliability of the application, minimize interaction with the patch, and decrease the risk of contamination to the patch.

Delivery of a patch using a patch application device in accordance with the methods of the present invention may involve acceleration of the patch application device itself to a desired velocity.

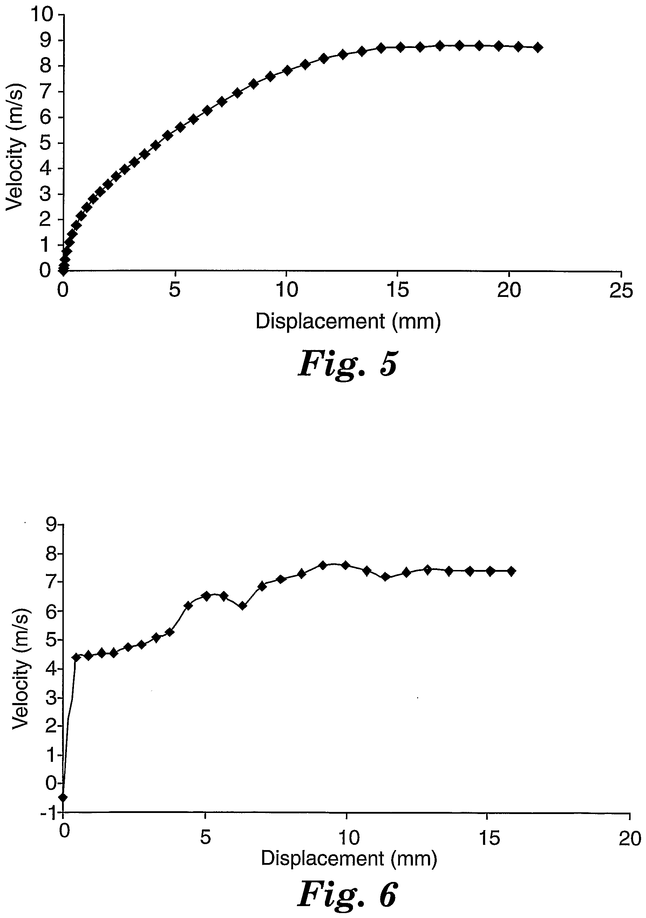

As discussed above, referring to Figure 5, a method of applying a patch using patch application devices of the present invention involves having the patch reach a desired velocity that is effective to pierce the microneedles through the stratum corneum layer of the skin. The desired velocity is, however, preferably controlled to limit or prevent stimulation of the underlying nerve tissue that would result in the sensation of pain, hematoma, or localized swelling. In connection with the present invention, the maximum velocity achieved by the patch and/or piston may preferably be 20 meters per second (m/s) or less, potentially 15 m/s or less, or possibly 10 m/s or less. In some instances, it may be more preferred that the maximum velocity be 8 m/s or less. At the lower end of the range of desired velocities, it may be preferred that the desired minimum velocity achieved by the patch and/or piston be 2 m/s or more, possibly 4 m/s or more, possibly more preferably 6 m/s or more.

Because of the variability in the location of skin, it may be preferred that the apparatus be designed such that the patch and/or piston travels at a velocity at or above the desired minimum velocities over a distance that is sufficient to accommodate the variations in skin location relative to the patch application device. For example, it might be preferred that the patch and/or piston in a patch application device moves at or above the minimum velocity over a distance of one millimeter or more. In some embodiments, it may be sufficient that the patch and/or piston move at or above the minimum velocity over a distance of 5 millimeters or more. Piston velocity of an application device as a function of displacement from the first stored position is shown in FIG. 5 for a device not containing a patch. Piston velocity (and patch velocity after the piston contacts the patch) of an application device as a function of displacement from the first stored position is shown in FIG. 6.

The force required to reach the desired velocities may vary based on the mass of the piston 42. That mass may also be controlled or selected to reduce the likelihood that nerve tissue underneath the delivery site is stimulated sufficiently to result in the sensation of pain. For example, the mass of the piston may be less than about 8 grams or less. It may be preferred that the mass of the piston be about 6 grams or less, more preferably about 4 grams or less.

The present invention also provides a kit that includes a cartridge for use with an applicator in a patch application device, a patch 20 within the collar characterized in that at least a portion of the distal-facing surface of the patch 20 contains an adhesive 24, and an applicator device having a first stored position and a second released position. The kit also provides instructions for attaching and detaching the collar 34 from the applicator device; and instructions for engaging the applicator from the first stored to the second released position and thereby applying the patch 20 to a target application surface 38, and packaging material to protect the applicator and the cartridge from environmental contamination. As contemplated herein, the kit is configured to store and provide at least one applicator device and cartridge in a ready-to-use condition. In one embodiment, the kit may include one applicator device and a plurality of cartridges, such that multiple treatments may be provided using a single kit. In one embodiment, the kit may include a jig or apparatus to assist in returning the application device from the second released position to the first stored position. Such a jig, for example, may be configured to assist in returning a piston to the first stored position when the device is in the second released position after application of a patch, preferably without having to manually handle the piston.

Patch application devices of the present invention are intended to deliver a patch which is intended to be left in place for a period of time during drug administration. As used in connection with the present invention, the term "microneedle" (and variations thereof) refers to structures having a height above the surface from which they protrude of about 500 micrometers or less.

Where the patch application devices are to be used for piercing the stratum corneum in preparation for transdermal drug delivery, the height of the microneedles is preferably sufficient to pass through the stratum corneum. It is also, however, preferable that the height of the microneedles is not sufficiently large to cause significant pain when inserted at a delivery site. In some instances, microneedles of the present invention may have a height of about 250 micrometers or less. In some instances, microneedles of the present invention may have a height of about 100 micrometers or more.

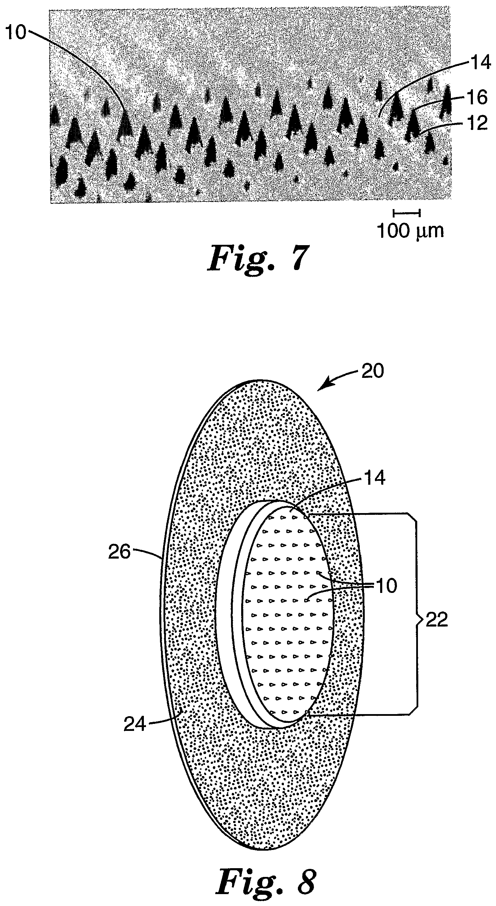

Referring to Figure 7, the general shape of the microneedles may be tapered. For example, the microneedles 10 have a larger base 12 at the substrate surface 14 and extend away from the substrate surface 14, tapering to a tip 16. In one embodiment the shape of the microneedles is pyramidal. In another embodiment, the shape of the microneedles is generally conical. In one embodiment the microneedles have a defined tip bluntness, such as that described in co-pending and commonly owned U.S. Patent Application Serial No. 10/621620, filed on July 17,2003 and entitled MICRONEEDLE DEVICES AND MICRONEEDLE DELIVERY APPARATUS (Attorney Docket No. 57901US005), wherein the microneedles have a flat tip comprising a surface area measured in a plane aligned with the base of about 20 square micrometers or more and 100 square micrometers or less. In one embodiment, the surface area of the flat tip will be measured as the cross-sectional area measured in a plane aligned with the base, the plane being located at a distance of 0.98h from the base, where h is the height of the microneedle above the substrate surface measured from base to tip.

The microneedles may preferably be manufactured integrally with the substrate. In other words, the various features may preferably be formed as a one piece, completely integral unit. Alternatively, the microneedles may be provided separately from the substrate.

Material selection may be based on a variety of factors including the ability of the material to accurately reproduce the desired pattern; the strength and toughness of the material when formed into the microneedles; the compatibility of the material with, for example, human or animal skin; the compatibility of the materials with any fluids that will be expected to contact the microneedle devices, etc.

Still another suitable microneedle construction comprises the structures described in United States Patent No. 6,091,975 (Daddona, et al.) which describes blade-like microprotrusions for piercing the skin. Still another microneedle construction is described in United States Patent No. 6,611,707 (Prausnitz, et al.).

Among polymeric materials, the microneedles are manufactured of thermoplastic polymeric materials. Suitable polymeric materials for the microneedles of the present invention may include, but are not limited to: acrylonitrile-butadiene-styrenes, polyphenyl sulfides, polycarbonates, polypropylenes, acetals, acrylics, polyetherimides, polybutylene terephthalates, polyethylene terephthalates, etc. Polymeric microneedles may be manufactured of a single polymer or a mixture/blend of two or more polymers.

Figure 8 illustrates a patch 20 according to one embodiment of the invention in the form of a combination of an array 22, pressure sensitive adhesive 24 and backing 26. A portion of the array 22 is illustrated with microneedles 10 protruding from a microneedle substrate surface 14. The microneedles 10 may be arranged in any desired pattern or distributed over the microneedle substrate surface 14 randomly. As shown, the microneedles 10 are arranged in uniformly spaced rows. In one embodiment, arrays of the present invention have a distal-facing surface area of more than about 0.1 cm² and less than about 20 cm², preferably more than about 0.5 cm² and less than about 5 cm². In the embodiment shown in FIG. 8, a portion of the substrate surface 16 is non-patterned. In one embodiment the non-patterned surface has an area of more than about 1 percent and less than about 75 percent of the total area of the device surface that faces a skin surface of a patient. In one embodiment the non-patterned surface has an area of more than about 0.10 square inch (0.65 cm²) to less than about 1 square inch (6.5 cm²). In another embodiment (not shown), the microneedles are disposed over substantially the entire surface area of the array 22.

In one embodiment, the patch 20 contains a pressure sensitive adhesive 24 on the distal-facing surface and a backing 26 on the proximal facing surface. Typical examples of flexible backings employed as conventional tape backings which may be useful for the present invention include those made from polymer films such as polypropylene; polyethylene, particularly low density polyethylene, linear low density polyethylene, metallocene polyethylenes, and high density polyethylene; polyvinyl chloride; polyester (e.g., polyethylene terephthalate); ethylene-vinyl acetate copolymer; polyurethane; cellulose acetate; and ethyl cellulose. Fabrics and non-wovens are also suitable. Coextruded multilayer polymeric films are also suitable, such as those described in U. S. Patent No. 5, 783, 269 (Heilmann et al.), the disclosure of which is incorporated herein by reference.

The backing 26 may be of thickness of about 0.010 inches (254 µm) or less, and more preferably about 0.005 inches (127 µm) or less, and even more preferably about 0.001 inches (25.4 µm). In some embodiments, the patch may comprise microneedles that may be coated with a drug formulation. Also, it should be noted that there is no requirement that the patches 20 be uniform, layered patches 20 such as those shown. For example, the patches may vary in size or shape and may include various structures, such as drug reservoirs.

It should be understood that the patch 20 is sufficiently flexible to allow for uniform adhesion to the application surface 38. Additionally, the patch is sufficiently flexible to bend around the obstructions in the collar thus preventing inadvertent contact between the patch and the obstructions. One skilled in the art will appreciate that the types of material used for the backing and pressure sensitive adhesive are sufficiently flexible. The patch may include other features, such as those described in U.S. Patent Application Serial No. 60/634,905, filed December 10, 2004 and entitled Medical Device (Attorney Docket No. 59760US002).

While the patch 20 is depicted essentially in a circular configuration, it will be appreciated that the patch 20 may be configured in any useful or ornamental configuration desired. It will further be appreciated that the description herein of the various embodiments of the invention are merely exemplary of patches that embody the principles of the present invention and that the described embodiments are not intended to be limitation on the broader concepts inherent in the described embodiments.

In one embodiment, by providing truncated tapered microneedles, patch application devices of the present invention may provide for effective penetration of, e.g., the stratum corneum, without stimulating the underlying nerve tissue that would result in the sensation of pain. As used herein, "effective penetration" means that the pathways opened through the stratum corneum by microneedles with larger tops may provide for enhanced transfer of materials through the stratum corneum. In addition, the tapered shape of the microneedles may enhance penetration of the stratum corneum as compared to microneedles with more column-like shapes that are not tapered.

Another manner in which the microneedles of patch application devices of the present invention may be characterized is based on the aspect ratio of the microneedles. As used herein, the term "aspect ratio" is the ratio of the height of the microneedle (above the surface surrounding the base of the microneedle) to the maximum base dimension, that is, the longest straight-line dimension that the base occupies (on the surface occupied by the base of the microneedle). In connection with the present invention, it may be preferred that the microneedles have an aspect ratio of 2:1 or higher, and in some instances 3:1 or higher.

In on embodiment, the microneedles used in connection with the present invention may have generally vertical wall angles, i.e. the microneedles may be in the form of pins, with sidewalls that are largely orthogonal to the surface of the substrate from which they protrude.

Although not depicted, the microneedle devices may include other features such as channels which are described in U.S. Patent Application Publication No. 2003-0045837. Furthermore, the microneedle devices may include covers pierced by the microneedles as described in U.S. Patent Publication No. 2003-0135161. Additionally, the microneedle arrays may be manufactured by several molding methods as described in co-pending U.S. Patent Application Serial No. 60/546,780, filed on February 23,2004 and entitled METHOD OF MOLDING FOR MICRONEEDLE ARRAYS.

Various modifications and alterations of this invention will become apparent to those skilled in the art without departing from the scope of this invention, and it should be understood that this invention is not to be unduly limited to the illustrative embodiments set forth herein.

## Claims

1. A patch application device (30) comprising:
a collar (34) comprising an inner and outer surface, wherein at least one obstruction (50) extends from the inner surface, and wherein the collar (34) further comprises a proximal end and a distal end;
a patch (20) within the collar (34) comprising a distal-facing surface wherein at least a portion of the distal-facing surface comprises an adhesive (24), which adheres the patch (20) to at least one obstruction (50);
an applicator adjacent to the proximal end of the collar (34), the applicator comprising:
a first stored position wherein at least a portion of the distal-facing surface of the patch (20) is protected from external contact;
a second released position wherein the patch (20) is released from contact with the at least one obstruction (50) in the collar (34) and expelled from the collar (34); and
a drive mechanism wherein the patch (20) is forcibly expelled toward the distal end of the collar (34);
wherein the patch (20) comprises a microneedle array (22) and backing (26), and
wherein the microneedle array (22) is manufactured of thermoplastic polymeric materials.

2. A device (30) according to claim 1, wherein the adhesive (24) comprises a pressure sensitive adhesive.

3. A device (30) according to claim 1, wherein the drive mechanism includes a piston (42), the piston (42) has a mass of about 2 grams or less.

4. A device (30) according to claim 1, wherein at least one obstruction (50) is coated with a release coating.

5. A patch application kit comprising:
the patch application device of claim 1 and at least one cartridge for use with the patch application device of claim 1, wherein the proximal end of the collar (34) is configured such that it may be attached and detached from the patch applicator;
instructions for attaching and detaching the collar (34) from the applicator; instructions for engaging the applicator from the first stored to the second released position and thereby applying the patch (20) to a target surface; and
packaging material to protect the applicator and at least one cartridge from contamination.

6. The patch application device (30) according to any one of claims 1 and 3, wherein the application device (30) is designed to drive the patch (20) at a minimum velocity of 4 meters per second or more.

7. The patch application device (30) according to any one of claims 1 and 3, wherein the application device (30) is designed to drive the patch (20) at a maximum velocity of 10 meters per second or less.

8. The patch application device (30) according to claim 1, wherein at least a portion of the microneedle array (22) comprises a non-patterned substrate.

9. The patch application device (30) according to any one of claims 1 and 8, wherein the array (22) comprises a surface area of 5 square centimeters or less.

10. The patch application device (30) according to claim 1, wherein the outer and inner surface of the collar (34) are coated with a release coating.

11. The patch application device (30) according to claim 1, wherein the inner surface is coated with a release coating.

## Patentansprüche

1. Pflasterapplikationsvorrichtung (30), aufweisend:
einen Kragen (34), der eine Innen- und eine Außenoberfläche aufweist, wobei mindestens ein Hindernis (50) von der Innenoberfläche aus vorsteht, und wobei der Kragen (34) ferner ein proximales Ende und ein distales Ende aufweist;
ein Pflaster (20) innerhalb des Kragens (34), das eine distal ausgerichtete Oberfläche aufweist, wobei zumindest ein Abschnitt der distal ausgerichteten Oberfläche ein Haftmittel (24) aufweist, welches das Pflaster (20) an mindestens einem Hindernis (50) anhaftet;
einen Applikator angrenzend an das proximale Ende des Kragens (34), wobei der Applikator aufweist:
eine erste Lagerposition, wobei zumindest ein Abschnitt der distal ausgerichteten Oberfläche des Pflasters (20) vor Außenkontakt geschützt ist;
eine zweite Freigabeposition, wobei das Pflaster (20) aus einem Kontakt mit dem mindestens einem Hindernis (50) in dem Kragen (34) gelöst ist und aus dem Kragen (34) ausgestoßen wird; und
einen Antriebsmechanismus, wobei das Pflaster (20) zwangsweise zum distalen Ende des Kragens (34) hin ausgestoßen wird;
wobei das Pflaster (20) ein Mikronadel-Array (22) und einen Träger (26) umfasst, und
wobei das Mikronadel-Array (22) aus thermoplastischen polymeren Materialien gefertigt ist.

2. Vorrichtung (30) nach Anspruch 1, wobei das Haftmitel (24) einen druckempfindlichen Kleber umfasst.

3. Vorrichtung (30) nach Anspruch 1, wobei der Antriebsmechanismus einen Kolben (42) beinhaltet, wobei der Kolben (42) eine Masse von etwa 2 Gramm oder weniger aufweist.

4. Vorrichtung (30) nach Anspruch 1, wobei mindestens ein Hindernis (50) mit einer Trennbeschichtung beschichtet ist.

5. Pflasterapplikationskit, umfassend
die Pflasterapplikationsvorrichtung nach Anspruch 1 und mindestens eine Patrone zur Verwendung mit der Pflasterapplikationsvorrichtung nach Anspruch 1, wobei das proximale Ende des Kragens (34) so gestaltet ist, dass es am Pflasterapplikator angebracht und davon gelöst werden kann;
Anweisungen zum Anbringen und Lösen des Kragens (34) vom Applikator;
Anweisungen zum Betätigen des Applikators aus der ersten Lagerposition in die zweite Freigabeposition und dadurch Applizieren des Pflasters (20) auf eine Zieloberfläche; und
Verpackungsmaterial, um den Applikator und mindestens eine Patrone vor Kontamination zu schützen.

6. Pflasterapplikationsvorrichtung (30) nach einem der Ansprüche 1 und 3, wobei die Applikationsvorrichtung (30) dafür ausgelegt ist, das Pflaster (20) mit einer Mindestgeschwindigkeit von 4 Metern pro Sekunde oder mehr anzutreiben.

7. Pflasterapplikationsvorrichtung (30) nach einem der Ansprüche 1 und 3, wobei die Applikationsvorrichtung (30) dafür ausgelegt ist, das Pflaster (20) mit einer Höchstgeschwindigkeit von 10 Metern pro Sekunde oder weniger anzutreiben.

8. Pflasterapplikationsvorrichtung (30) nach Anspruch 1, wobei zumindest ein Abschnitt des Mikronadel-Array (22) ein ungemustertes Substrat umfasst.

9. Pflasterapplikationsvorrichtung (30) nach einem der Ansprüche 1 und 8, wobei das Array (22) eine Oberfläche von 5 Quadratzentimetern oder weniger umfasst.

10. Pflasterapplikationsvorrichtung (30) nach Anspruch 1, wobei die Außen- und die Innenoberfläche des Kragens (34) mit einer Trennbeschichtung beschichtet sind.

11. Pflasterapplikationsvorrichtung (30) nach Anspruch 1, wobei die Innenoberfläche mit einer Trennbeschichtung beschichtet ist.

## Revendications

1. Dispositif d'application de timbre (30) comprenant :
un collier (34) comprenant une surface interne et une surface externe, dans lequel au moins une obstruction (50) s'étend à partir de la surface interne, et dans lequel le collier (34) comprend en outre une extrémité proximale et une extrémité distale ;
un timbre (20) au sein du collier (34), comprenant une surface à orientation distale dans lequel au moins une partie de la surface à orientation distale comprend un adhésif (24), qui fait adhérer le timbre (20) à au moins une obstruction (50) ;
un applicateur adjacent à l'extrémité proximale du collier (34), l'applicateur comprenant :
une première position rangée, dans lequel au moins une partie de la surface à orientation distale du timbre (20) est protégée d'un contact externe ;
une deuxième position libérée, dans lequel le timbre (20) est libéré d'un contact avec l'au moins une obstruction (50) dans le collier (34) et expulsé du collier (34) ; et
un mécanisme d'entraînement, dans lequel le timbre (20) est expulsé de force en direction de l'extrémité distale du collier (34) ;
dans lequel le timbre (20) comprend un réseau de micro-aiguilles (22) et un support (26), et
dans lequel le réseau de micro-aiguilles (22) est fabriqué à partir de matériaux polymères thermoplastiques.

2. Dispositif (30) selon la revendication 1, dans lequel l'adhésif (24) comprend un adhésif sensible à la pression.

3. Dispositif (30) selon la revendication 1, dans lequel le mécanisme d'entraînement inclut un piston (42), le piston (42) a une masse d'environ 2 grammes ou moins.

4. Dispositif (30) selon la revendication 1, dans lequel au moins une obstruction (50) est revêtue d'un revêtement de libération.

5. Trousse d'application de timbre, comprenant :
le dispositif d'application de timbre selon la revendication 1 et au moins une cartouche destinée à être utilisée avec le dispositif d'application de timbre selon la revendication 1, dans laquelle l'extrémité proximale du collier (34) est configurée de telle sorte qu'elle peut être fixée à l'applicateur de timbre et détachée de celui-ci ;
des instructions pour fixer et détacher le collier (34) par rapport à l'applicateur ;
des instructions pour amener l'applicateur de la première position rangée à la deuxième position libérée et appliquer de ce fait le timbre (20) sur une surface cible ; et
un matériau d'emballage pour protéger l'applicateur et au moins une cartouche de la contamination.

6. Dispositif d'application de timbre (30) selon l'une quelconque des revendications 1 et 3, dans lequel le dispositif d'application (30) est conçu pour entraîner le timbre (20) à une vitesse minimale de 4 mètres par seconde ou plus.

7. Dispositif d'application de timbre (30) selon l'une quelconque des revendications 1 et 3, où le dispositif d'application (30) est conçu pour entraîner le timbre (20) à une vitesse maximale de 10 mètres par seconde ou moins.

8. Dispositif d'application de timbre (30) selon la revendication 1, dans lequel au moins une partie du réseau de micro-aiguilles (22) comprend un substrat sans motif.

9. Dispositif d'application de timbre (30) selon l'une quelconque des revendications 1 et 8, dans lequel le réseau (22) comprend une superficie de 5 centimètres carrés ou moins.

10. Dispositif d'application de timbre (30) selon la revendication 1, dans lequel les surfaces externe et interne du collier (34) sont revêtues d'un revêtement de libération.

11. Dispositif d'application de timbre (30) selon la revendication 1, dans lequel la surface interne est revêtue d'un revêtement de libération.
